# EUROPEAN PATENT APPLICATION

(11) **EP 1 762 244 A1**
(43) Date of publication of application: **14.03.2007**
(21) Application number: 06020758.6
(22) Date of filing: 07.09.2000
(51) Int. Cl.: A61K 38/18, A61K 35/30, A61P 25/28, C12N 5/06

(54) **BMP-9 COMPOSITIONS FOR INDUCING DIFFERENTIATION OF CHOLINERGIC NEURONS**

(30) Priority: 08.09.1999 US 153121 P
(62) Divisional of application: 00961631.9
(71) Applicant: Genetics Institute, LLC, Cambridge, MA 02140 (US); Trustees of Boston University, Boston, MA 02215 (US)
(72) Inventor: Blusztajn, Jan, Krzysztof, Cambridge, MA 02140 (US); Lopez-Coviella, Ignacio, Brighton, MA 02135 (US); Krauss, Raul, Newton, MA 02458 (US); Thies, R. Scott, Pleasanton, CA 94566 (US)
(74) Representative: Vossius & Partner

(57) **Abstract**

The present Invention describes the use of an effective amount of a BMP-9 protein for the preparation of a medicament for treating degenerating and/or malfunctioning cholinergic neurons in a patient in need of same wherein the medicament is used to upregulate the genes for choline acetyltransferase and/or vesicular acetylcholine transporter to induce the cholinergic phenotype.

## Description

### GOVERNMENT SUPPORT

This invention was made with Government Support under Contract Number P01AG09525 awarded by the National Institute on Aging and Contact Number IBN-9907572 awarded by the National Science Foundation. The Government has certain rights in the invention.

### BACKGROUND OF THE INVENTION

The present invention relates to BMP-9 pharmaceutical compositions and methods for using them for the induction and maintenance of the cholinergenic phenotype of the central nervous system (CNS). These compositions and methods may be used treat CNS diseases or disorders involving cholinergic neurons.

BMP-9 DNA sequences, composition and methods for producing them are disclosed in PCT publication WO 93/00432 the disclosure of which is incorporated herein by reference. BMPs promote the survival and phenotypic maturation of neurons in the peripheral nervous system and lineage-restricted neuronal progenitor cells in the CNS. Cholinergic neurons synthesize, store and release acetylcholine (ACh). By the present invention it has been discovered that BMP-9, synthesized locally in the vicinity of the developing cholinergic neurons, acts to induce the expression of the cholinergic gene locus in these cells. BMP-9 expressed in the embryonic mouse septum and spinal cord induces the cholinergic neurotransmitter phenotype in cultured embryonic mouse CNS neurons. BMP-9 compositions of the invention may be used as a cholinergic differentiation factor in the treatment of diseases that affect cholinergic neurons. Depending on brain region and developmental stage, various BMPs may regulate the expression of other neurotransmitter phenotypes.

The invention therefore provides pharmaceutical compositions containing a therapeutically effective amount of a BMP-9 protein in a pharmaceutically acceptable vehicle or carrier. The cholinergic neurons of the basal forebrain are understood to be important for cognition and memory and undergo severe degeneration in Alzheimer's disease. In one embodiment, BMP-9 compositions of the invention may therefore be used in the treatment of degenerating cholinergic neurons in Alzheimer's disease. BMP-9 compositions may also be used to treat other diseases/disorders involving degenerating and/or malfunctioning cholinergic neurons.

BMP-9 may be used as a motor neuron factor. Therefore BMP-9 may be used for the treatment of such conditions as amyotrophic lateral sclerosis (Lou Gehrig disease).

BMP-9 compositions may be further characterized by the ability to demonstrate effects upon the growth and/or differentiation of embryonic and/or stem cells. Therefore, in another embodiment the BMP-9 compositions of the invention may be used in cell replacement therapy in which embryonic basal forebrain cells are treated in vitro to generate cholinergic neurons before transplantation into patients.

Compositions of the invention may further include at least one other therapeutically useful agent such as the BMP proteins BMP-1, BMP-2, BMP-3, BMP-4, BMP-5, BMP-6 and BMP-7, disclosed for instance in United States Patents 5,108,922; 5,013,649; 5,116,738; 5,106,748; 5,187,076; and 5,141,905; BMP-8, disclosed in PCT publication WO91/18098; BMP-10, disclosed in PCT application WO94/26893; BMP-11, disclosed in PCT application WO94/26892, or BMP-12 or BMP-13, disclosed in PCT application WO 95/16035, or BMP-15, disclosed in copending patent application, serial no. 08/446,924, filed on May 18, 1995.

The compositions of the invention may comprise, in addition to a BMP-9 protein, other therapeutically useful agents including growth factors such as epidermal growth factor (EGF), fibroblast growth factor (FGF), transforming growth factor (TGF-α and TGF-β), activins, inhibins and insulin-like growth factor (IGF).

In certain applications, as determined by one skilled in the art, the compositions may also include an appropriate matrix for example, for supporting the composition. The matrix may provide slow release of the protein and/or the appropriate environment for presentation thereof.

The BMP-9 compositions may be employed in methods for treating a number of neuronal diseases and/or defects. These methods, according to the invention, entail administering to a patient needing treatment, such as degenerating neurons for example as in Alzheimer's, an effective amount of a BMP-9 protein composition. Another method entails cell replacement therapy wherein embryonic basal forebrain cells are treated in vitro with BMP-9 to generate cholinergic neurons before transplantation into patients for example, Alzheimer's patients or other patients requiring differentiation and/or maintenance of cholinergenic neurons.

The BMP-9 DNA sequences are used, in another embodiment, for preparing vectors for gene therapy applications whereby BMP-9 is delivered by implanted cells transfected with the BMP-9 gene. In such use, the vectors may be transfected into the cells of a patient *ex vivo,* and the cells may be reintroduced into a patient. Alternatively, the vectors may be introduced into a patient *in vivo* through targeted transfection.

These methods may also entail the administration of a protein of the invention in conjunction with at least one of the novel BMP proteins disclosed in the co-owned applications described above. In addition, these methods may also include the administration of a BMP-9 protein with other growth factors including EGF, TGF-*a,* TGF-*b,* FGF and IGF.

Other aspects and advantages of the present invention will be apparent upon consideration of the following detailed description and preferred embodiments thereof.

### BRIEF DESCRIPTION OF THE DRAWINGS

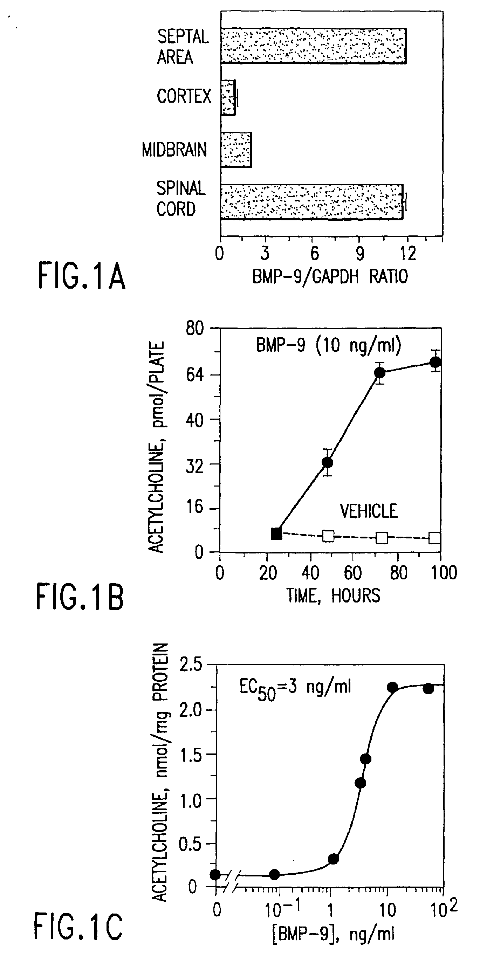

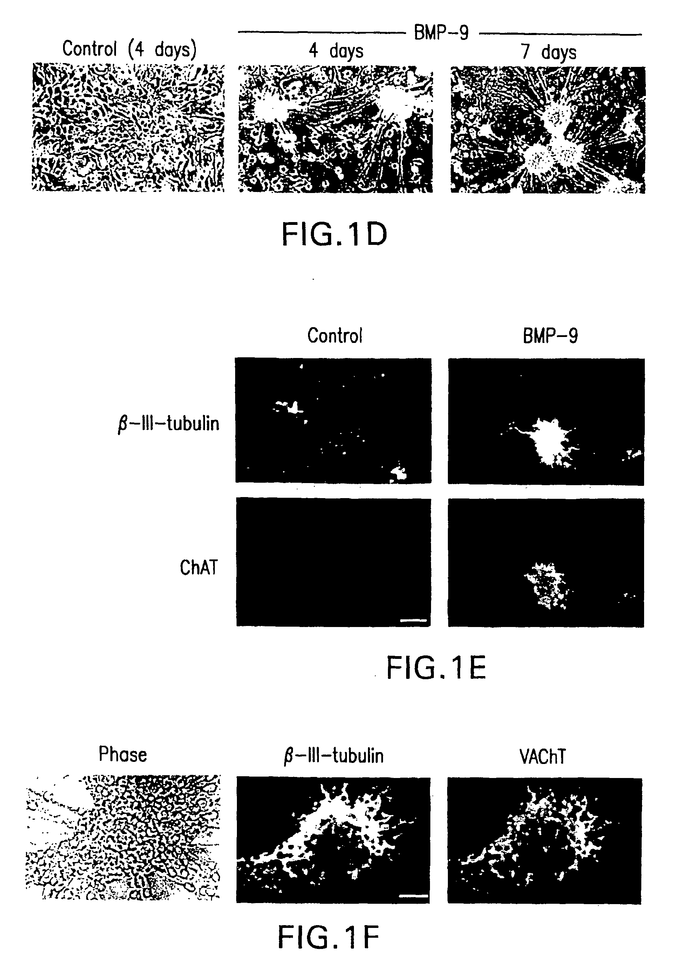

FIG. 1 sets forth results of studies of the expression of BMP-9 in mouse embryonic CNS, and induction of the cholinergic phenotype in cultured mouse basal forebrain neurons. FIG. 1a) sets forth the results of the mRNA levels of BMP-9 measured by real time quantitative RT-PCR in E14 brain regions indicated. Total RNA was prepared from a pool of at least 10 animals in each age group. FIG. 1 (b-c) sets forth results of E 14 septal cultures treated with BMP-9 for varied time periods (b), or for three days with varied concentrations of BMP-9 (c), with measurement of acetylcholine. Values are means +/- SEM (n=3) (b) and means (n=2) (c); FIG. d-f) sets forth results from E14 septal cultures treated for four days with BMP-9 (10 ng/ml) with the cells examined by phase contrast (d,f), double immunofluorescence staining with anti-β-III-tubulin antibodies (e,f) combined with anti-ChAT (e), or anti-VAChT antibodies (f). Scale bars: 100 micrometers, d,e; 50 Fm, f.

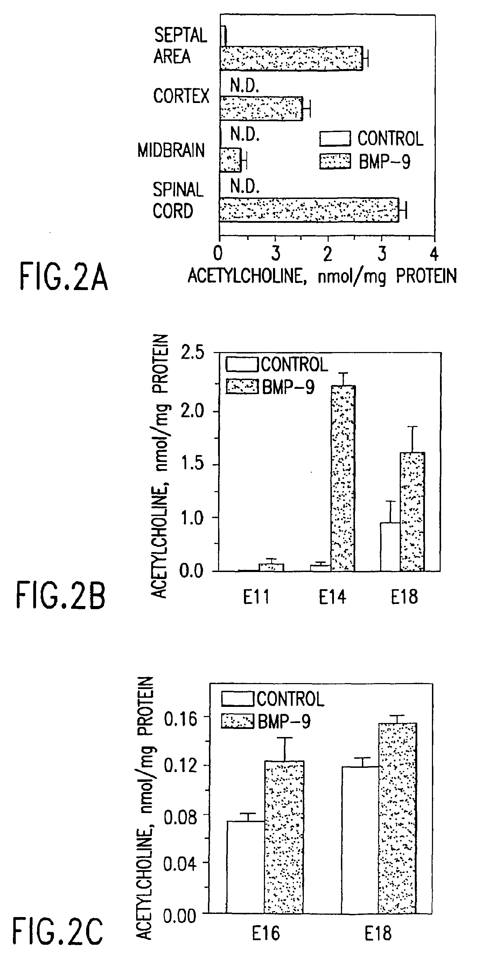

FIG. 2a and 2b set forth results of the study of BMP-9 induction of the expression of the cholinergic phenotype in a fashion specific for brain region and developmental stage. The cultures were treated for three days with 10 ng/ml of BMP-9 and ACh was measured. FIG. 2a) sets forth results of brain region specificity in E14 cultures from the CNS regions. FIG. 2b) sets forth results of developmental stage specificity in Septal area cultures obtained at E11, E14, and E18. Fig 2c sets forth advanced maturation of the cholinergic phenotype *in vivo.* Mouse embryos were injected intracerebroventricularly with 6ng of BMP-9 and brain tissue was processed for Ach content two days after. Values are means +/- SEM (n = 5).

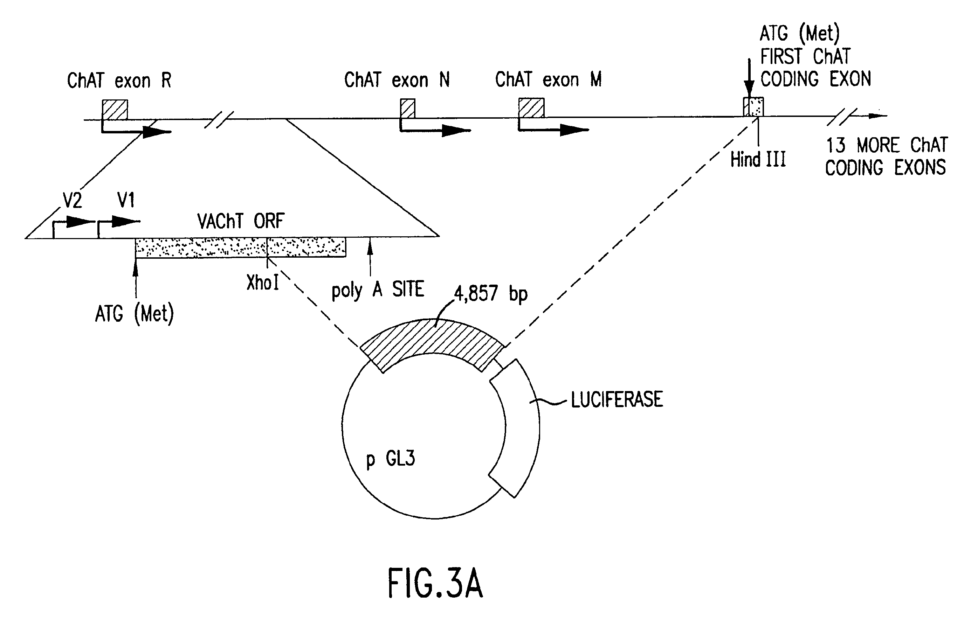

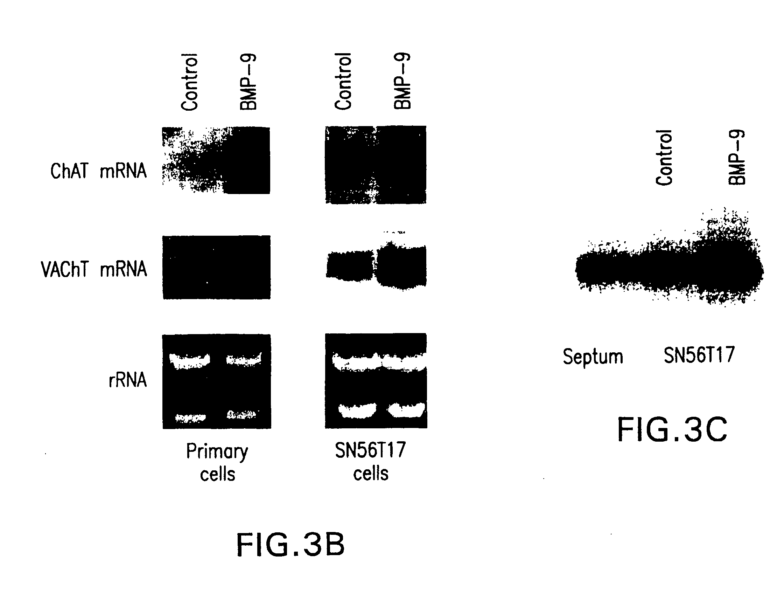

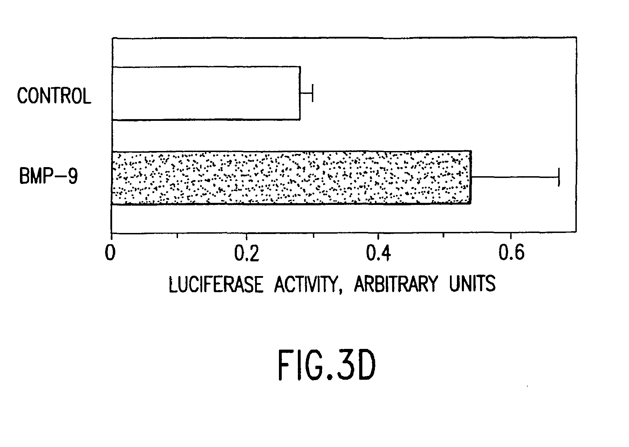

FIG. 3 sets forth results of the studies regarding BMP-9 induction of the expression of the cholinergic gene locus. FIG. 3a) sets forth organization of the cholinergic gene locus and the reporter gene construct used in panel d. The noncoding exons R, N, and M are shown as black boxes and the coding sequences are in gray. The intronless open reading frame of VAChT resides between exons R and N of ChAT. For the reporter gene assays the 4,857 bp XhoI-HindIII fragment of the murine gene was inserted into the pGL3 basic luciferase plasmid. FIG. 3b) sets forth results of E14 septal cultures or SN56T17 cells treated with 10 ng/ml of BMP-9 for three days and ChAT and VAChT mRNA was determined by Northern blotting. FIG. 3c) sets froth results of the study of BMP-9 upregulation of the expression of the M exon of ChAT. SN56T17 cells were treated for two days with 10 ng/ml of BMP-9 and the expression of the M exon was determined by RT-PCR. Adult mouse septum was used as a positive control. FIG. 3d) sets forth the results of the upregulation of luciferase expression driven by the ChAT promoter in SN56T17 cells treated for two days with 10 ng/ml BMP-9.

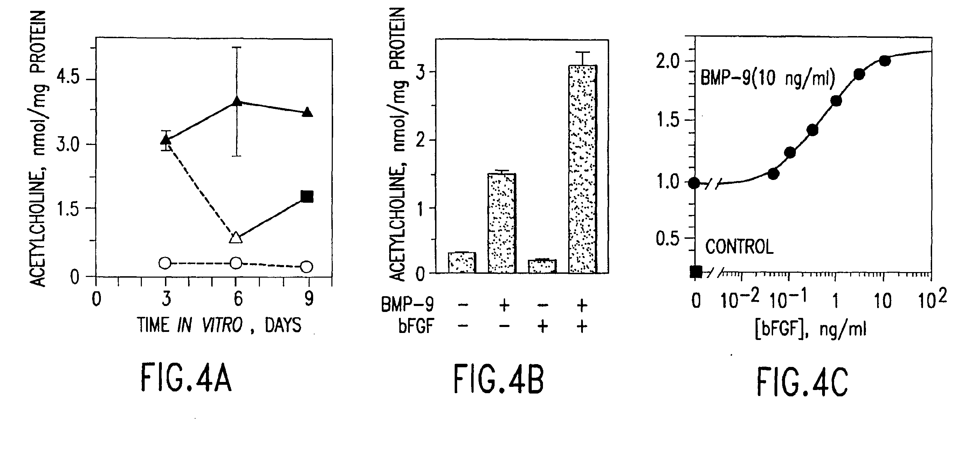

FIG. 4 sets forth the results of the studies of BMP-9 in the maintenance of the induced cholinergic phenotype and FGF potentiation of the induction of the cholinergic phenotype evoked by BMP-9. FIG. 4a) sets forth the results of ACh measured in septal cultures from E14 mice treated with 10 ng/ml of BMP-9 (solid triangles) for 3, 6, and 9 days; for 3 days following a 3 day withdrawal of BMP-9 (open triangle), and for an additional 3 day period with BMP-9 (filled square). Controls received no BMP-9 (open circles). FIG. 4 (b-c) sets forth results of E14 septal cultures treated for three days with the factors indicated (b), BMP-9 (10 ng/ml); bFGF (10 ng/ml), or (c) in the presence of BMP-9 with varying concentrations of bFGF, and ACh measurement. The values are means ± s.d.

### DETAILED DESCRIPTION OF THE INVENTION

BMP-9 DNA sequences, proteins and methods for their production have been disclosed in WO 93/00432 incorporated herein by reference. By the present invention it has been discovered that BMP-9 induces and maintains the cholinergic phenotype of neurons in the CNS. More particularly, the present invention demonstrates that BMP-9 is highly expressed in the embryonic mouse septum and spinal cord and induces the cholinergic neurotransmitter phenotype in cultured embryonic mouse CNS neurons. BMP-9 acts directly by inducing the expression of the cholinergic gene locus encoding choline acetyltransferase and the vesicular acetylcholine (ACh) transporter, resulting in an upregulation of ACh synthesis. Withdrawal of BMP-9 reduces ACh synthesis indicating that in addition to inducing the cholinergic phenotype, BMP-9 is necessary for its maintenance.

A BMP-9 composition of the present invention, which induces differentiation of and/or malfunctioning of cholinergic neurons, has application in the treatment of conditions exhibiting a degeneration of cholinergic neurons and/or requiring maintenance thereof. An example of a condition which may be treated with BMP-9 compositions of the present invention is Alzheimer's. BMP-9 compositions of the invention may be further characterized by the ability to demonstrate effects upon the growth and/or differentiation of embryonic cells and/or stem cells. The proteins or compositions of the present invention may also be useful for treating cell populations, such as embryonic cells or stem cell populations, to enhance or enrich the growth and/or differentiation of the cells. The BMP-9 compositions may also provide valuable component of cell culture media.

Another aspect of the present invention provides novel methods for administering BMP-9 compositions. One method involves treatment of the patient needing differentiation and/or maintenance of cholinergic neurons. In another embodiment involving cell replacement therapy, BMP-9 is added to the embryonic basal forebrain cells in vitro to generate cholinergic neurons them transplanted into patients.

Such compositions comprise a therapeutically effective amount of a BMP-9 proteins of the invention in admixture with a pharmaceutically acceptable vehicle, carrier or matrix. It is expected that the proteins of the invention may act in concert with or perhaps synergistically with other related proteins and growth factors. Further therapeutic methods and compositions of the invention therefore comprise a therapeutic amount of at least one BMP-9 protein of the invention with a therapeutic amount of at least one of the other BMP proteins, BMP-1, BMP-2, BMP-3, BMP-4, BMP-5, BMP-6 and BMP-7, disclosed for instance in United States Patents 5,108,922; 5,013,649; 5,116,738; 5,106,748; 5,187,076; and 5,141,905; BMP-8, disclosed in PCT publication WO91/18098; BMP-10, disclosed in PCT application WO94/26893; BMP-11, disclosed in PCT application WO94/26892, BMP-12 or BMP-13, disclosed in PCT application WO 95/16035, or BMP-15, disclosed in copending patent application, serial no. 08/446,924, filed on May 18, 1995. Other compositions which may also be useful include Vgr-2, and any of the growth and differentiation factors [GDFs], including those described in PCT applications WO94/15965; WO94/15949; WO95/01801; WO95/01802; WO94/21681; WO94/15966; WO95/10539; WO96/01845; WO96/02559 and others. Also useful in the present invention may be BIP, disclosed in WO94/01557; HP00269, disclosed in JP Publication number: 7-250688; and MP52, disclosed in PCT application WO93/16099. The disclosures of the above applications are hereby incorporated by reference herein. Such combinations may comprise separate molecules of the BMP proteins or heteromolecules comprised of different BMP moieties. For example, a method and composition of the invention may comprise a disulfide linked dimer comprising a BMP-9 protein subunit and a subunit from one of the "BMP" proteins described above. A further embodiment may comprise a heterodimer of BMP-9 moieties. These agents include various growth factors such as epidermal growth factor (EGF), fibroblast growth factor (FGF), platelet derived growth factor (PDGF), transforming growth factors (TGF-α and TGF-β), activins, inhibins, and k-fibroblast growth factor (kFGF), parathyroid hormone (PTH), parathyroid hormone related peptide (PTHrP), leukemia inhibitory factor (LIF/HILA/DA), insulin-like growth factors (IGF-I and IFG-II). Portions of these agents may also be used in compositions of the present invention.

The method of administration of the composition depends, of course, on the method of treatment. For the treatment of cholinergic neurons in Alzheimer's patients, BMP-9 compositions could be delivered directly to the brain by injection either intraparenchymally, intraventricularly into the ventricle spaces of the brain, or intrathecally into the cerebrospinal fluid. Methods involving cell replacement therapy would involve treating cells in vitro before transplantation into patients. In gene therapy applications BMP-9 is delivered by implanted cells transfected with the BMP-9 gene. When administered, the therapeutic composition for use in this invention is, of course, in a pyrogen-free, physiologically acceptable form. Further, the composition may desirably be encapsulated or injected in a viscous form for delivery to the site of neuronal degeneration or site where differentiation is needed. Therapeutically useful agents other than the BMP-9 proteins which may also optionally be included in the composition as described above, may alternatively or additionally, be administered simultaneously or sequentially with the BMP composition in the methods of the invention. In certain applications a matrix may be desired. The matrix may provide slow release of BMP-9 and/or the appropriate environment for presentation thereof. Such matrices may be formed of materials presently in use for other implanted medical applications.

The preparation and formulation of such physiologically acceptable protein compositions, having due regard to pH, isotonicity, stability and the like, is within the skill of the art. Pharmaceutical compositions suitable for use in the method of the present invention may contain, in addition to the BMP, pharmaceutically acceptable carriers, dilutents, fillers, salts, buffers, stabilizers, and/or other materials well known in the art. The term "pharmaceutically acceptable" means a non-toxic material that does not interfere with the effectiveness of the biological activity of the active ingredients(s). The characteristics of the carrier of other material will depend on the route of administration.

The dosage regimen will be determined by the attending physician considering various factors which modify the action of the BMP-9 protein, e.g., amount of differentiation desired, the nature and condition being treated, the patient's age, sex, and diet, the severity of any infection, time of administration and other clinical factors. Generally, systemic or injectable administration will be initiated at a dose which is minimally effective, and the dose will be increased over a preselected time course until a positive effect is observed. Subsequently, incremental increases in dosage will be made limiting such incremental increases to such levels that produce a corresponding increase in effect, while taking into account any adverse affects that may appear. The addition of other known growth factors, such as IGF I or FGF, to the final composition, may also effect the dosage.

The term "therapeutically effective amount" means the total amount of each active component of the method that is sufficient to show a meaningful patient benefit, i.e., healing of chronic conditions or increase in rate of healing. Progress can be monitored by periodic assessment of the patient as determined by the attending physician.

The following examples illustrate practice of the present invention in determining a novel developmental function for BMP-9 and employing it to induce the expression of the cholinergic gene locus in these cells and its use in the treatment of diseases that affect cholinergic neurons.

### EXAMPLE I

### Cell Culture and Embryo Injection

Primary neuronal cultures were prepared by a modification of the method described by Hartikka and Hefti (Hartikka, J. and Hefti, F. J.Neurosci. 8, 2967-2985, 1988) for basal forebrain cholinergic neurons. Dissociated cells, obtained from the septal area of embryonic day 11-18 mice (Schambra, U. B., Lauder, J. M., and Silver, J. Atlas of the prenatal mouse brain. 1992. San Diego, Academic Press) were plated on poly-L-lysine/laminin-coated tissue culture dishes and incubated with Dulbecco Modified Eagle Medium (DMEM) containing 10% heat inactivated fetal bovine serum and basic fibroblast growth factor (bFGF) (20 ng/ml). BMP-9 and other TGF-$ family members (Genetics Institute, Inc.) were added immediately after plating and every 24 hours until the cells were collected for analysis. Transfections and reporter plasmid experiments were carried out on mouse cholinergic septal SN56T17 cells (Hammond, D.N., Wainer, B.H., Tonsgard, J.H., and Heller, A. Science 234, 1237-1240, 1986; Lee, H.J., Hammond, D.N., Large, T.H., and Wainer, B.H. Dev.Brain Res. 52, 219-228 1990). The cells were maintained in DMEM medium containing 10% FBS, and 50 Fg/ml gentamicin.

Intracerebroventricular injections (1µl) of BMP-9 (6ng) and vehicle, both containing bFGF (20ng), were performed in embryos at E14 and E16. Dams (and embryos) were anesthesized with isofluorane. Following a midventral laparotomy, the uterus was extemalized and the embryos were injected through the uterine wall, into the ventricular system, by means of a 33-gauge needle attached to a microcannula. This procedure was carried out under a surgical microscope. Following the injections, the uterus was repositioned into the abdominal cavity, which was closed at various planes with absorbable and nonabsorbable surgical suture. Animals were allowed to recover and the sacrificed two days later by CO 2 inhalation. Brain tissue from the embryos was collected, and Ach content of the forebrain was measured.

### EXAMPLE II

### BMP 9 Function in the CNS

The possible function of BMP-9 in the CNS, was determined by the expression of BMP-9 during mouse development using quantitative RT-PCR. BMP-9 mRNA was measured in the septum, cortex, mesencephalon and spinal cord dissected from E14 mice and snap frozen in liquid nitrogen. Total RNA was prepared using a Quiagen RNeasy Mini Kit. To avoid animal to animal variations, each sample contained RNA prepared from a pool of at least 10 animals. cDNA was prepared using a Superscript Preamplification System (GIBCO BRL, Gaithersburg MD). Real time semi-quantitative PCR was performed using the SYBR Green PCR Reagents and an ABI PRISM 7700 Sequence Detection System (PE Applied Biosystems, Foster City CA). BMP-9 mRNA levels were normalized to levels of GAPDH. The PCR primers used were: BMP9, forward primer: 5'-TCCCCACCGACTTGTTCTTC-3', reverse primer: 5'-GAGAGTCAGCTGGGAGCTTGA-3'. GAPDH, forward primer: 5'-TGTGTCCGTCGTGGATCTGA-3', reverse primer: 5'-CCTGCTTCACCACCTTCTTGA-3'. RT-PCR for the M exon of ChAT was performed using the Access RT-PCR system from Promega. The forward primer (5'-GGG GTG GCT GGT TTG CTT GCA GTC A -3') was designed specifically for detection of transcripts originating at the M promoter, and the reverse primer (5'-GGG GGC ACT GGC AAC TTA GGT AAG -3') was derived from the coding region of the ChAT gene. Following PCR, amplification products were subjected to Southern blotting with a ChAT-specific probe and visualized by autoradiography. To make a reporter construct, a 4.8-kb XhoI-HindIII fragment of the ChAT promoter (a gift from Dr. Jorge Naciff) was inserted upstream of the luciferase coding region in the plasmid pGL3 from Promega. SN56T17 cells were transfected using LipofectAMINE (Gibco) and luciferase activity was measured with the Luciferase Assay System from Promega.

On embryonic day 14 (E14) the highest abundance of BMP-9 mRNA was found in the septum and spinal cord (Fig. 1a). Based on the resemblance of this expression pattern to that of mature cholinergic neurons it was contemplated that BMP-9 may influence the development of these cells. To test this hypothesis, primary cells derived from the septal area of E14 mice were treated with human recombinant BMP-9 and measured ACh in the cultures (Fig. 1b, c). Ach in the cultures was measured by incubating cells for one hour at 37°C in physiological salt solution supplemented with 15 µM choline and 15 µM neostigmine (Berse, B. and Blusztajn, J.K. J.Biol.Chem. 270, 22101-22104, 1995; Berse, B., López-Coviella, I., and Blusztajn, J.K. Biochem.J. 342, 301-308, 1999). The cells were washed once with ice-cold choline-free physiological salt solution supplemented with 15 µM neostigmine. The cells were collected in methanol, centrifuged, and ACh was extracted from the methanol in 1 N formic acid, chloroform and water (1:0.1:2:1 by volume). The aqueous phase was collected, dried under a vacuum, and reconstituted in water. The content of ACh was determined by HPLC with an enzymatic reactor containing acetylcholinesterase and choline oxidase in series with an electrochemical detector (Bioanalytical Systems, Inc.) based on the method of Potter et al.(Potter, P.E., Meek, J.L., and Neff, N.H. J.Neurochem. 41, 188-194, 1983). To maintain a level of precursor cell division and differentiation similar to that observed in vivo (Li, W., Cogswell, C.A., and LoTurco, J.J. J Neurosci 18, 8853-8862 1998), the culture medium contained 10% of fetal bovine serum, and basic fibroblast growth factor (bFGF; 20 ng/ml). BMP-9 increased ACh content of these cells in a time-(Fig. 1b) and concentration-dependent (Fig. 1c) fashion. After an initial 24 hour lag period following the addition of BMP-9 (10 ng/ml), ACh levels rose monotonically until 72 h and then tended to level off. Untreated cultures maintained their ACh content throughout the study, indicating that cholinergic neurons initially present in the cultures did not degenerate in the absence of BMP-9. In accordance with this data BMP-9 upregulates the cholinergic phenotype of cholinergic cells or of cells committed to become cholinergic, or acts as an instructive factor for uncommitted neuronal progenitor cells to develop as cholinergic neurons. The amounts of Ach produced in the BMP-9-treated cultures are similar to those observed in adult mouse brain in situ.

To determine the specificity of BMP-9 action the cells were treated for 96 hours with additional members of the BMP family of proteins. In the absence of BMPs, ACh levels were low (6.4 ± 1.3 pmol/plate) and the addition of BMPs (10 ng/ml) increased the levels of this neurotransmitter to varied extents. BMP-6, BMP-7 and BMP-12 caused a 5-fold increase in cellular ACh levels. BMP-2 and BMP-4 increased ACh levels 13- and 14- fold, respectively. BMP-9 was the most effective among the factors tested, increasing the cellular ACh content 20- fold. Moreover, this effect of BMPs was not shared by TGF-β which did not significantly affect the levels of Ach. Therefore, BMP-2 and BMP-4, as well as BMPs 6, 7 and 12 may also be useful in compositions and methods for the induction and maintenance of the cholinergic phenotype of the CNS.

### EXAMPLE III

### Morphology of Neural Cultures

In addition to the above-described neurochemical effects, BMP-9 alters the morphology of neural cultures. Whereas untreated cells grow in uniformly-dispersed monolayers, cells exposed to BMP-9 tended to grow in characteristic round clusters and extended long and numerous processes (Fig. 1d).

Immunofluorescence studies were performed using goat affinity-purified polyclonal antibodies for ChAT (1:100), TH (1:200), GAD (1:1000) and VAChT (1:500), and a mouse monoclonal antibody against β-III-tubulin (1:500) (all from Chemicon), done in parallel with negative and positive controls. Cells plated on Biocoat chamber slides (Falcon) or cover-glass coated with poly-D-lysine and laminin were fixed in methanol for 5 minutes at -10°C and incubated at room temperature with 0.1% Triton-100 solution. The cells were washed with PBS, blocked for 30 min with 1% BSA, incubated overnight with primary antibodies in 1 % BSA and for 40 minutes with the appropriate fluorescent Alexa-conjugated secondary antibodies (Molecular Probes). Slides were mounted using Prolong Antifade (Molecular Probes) and visualized on an epifluorescence equipped microscope.

Double label immunofluorescence studies revealed that these neuronal clusters were positive for β-III-tubulin (an early marker for neurons during development (Menezes, J. and Luskin, M. J Neurosci 14, 5399-5416, 1994) and concurrently also expressed choline acetyltransferase (ChAT), the ACh synthesizing enzyme (Fig. 1e). Neuronal labeling of untreated cells (β-III-tubulin positive controls) was found, however, ChAT immunofluorescence was almost completely absent (Fig.1e). To further investigate the identity of the neuronal clusters generated by treatment with BMP-9, double label experiments were repeated with antibodies against β-III-tubulin and the vesicular acetylcholine transporter (VAChT) protein, a cholinergic marker. In most cases, the β-III-tub-positive neurons in these clusters were also positively stained for VAChT (Fig. 1f). Parallel immunofluorescence experiments with antibodies against tyrosine hydroxylase (TH) and glutamic acid decarboxylase (GAD) B markers for catecholaminergic and GABAergic neurons, respectively - revealed no positive staining for these proteins in cells treated with BMP-9, and only a slight increase in TH immunoreactivity was observed by Western blot. These data suggest that BMP-9 specifically promotes the cholinergic, but not the catecholaminergic or GABAergic, phenotype of neural cells from the septal area. The greatest responsiveness to BMP-9 occurred in cells derived from brain regions that abundantly express BMP-9 (Fig. 1a) and contain high numbers of cholinergic neurons, namely spinal cord and septum (Fig. 2a). In contrast, cultures of midbrain and cerebral cortex responded less well to BMP-9 (Fig. 2a).

### EXAMPLE IV

### Stage of Development

Determination of cell fate by extrinsic factors may be dependent on the stage of development. Cholinergic neurons are among the first to leave the mitotic cycle in the mouse basal forebrain (Schambra, U.B., Sulik, K.K., Petrusz, P., and Lauder, J.M., J.Comp. Neurol. 288, 101-122, 1989) and cholinergic neurogenesis begins in a caudo-rostral progression from E11 until E18 (Schambra, U.B., Sulik, K.K., Petrusz, P., and Lauder, J.M., J.Comp. Neurol. 288, 101-122, 1989; Semba, K. and Fibiger, J.Comp.Neurol. 269, 87-95, 1988; Sweeney, J. E., Hohmann, C. F., Oster-Granite, M. L., and Coyle, J. T., Neuroscience 31, 413-425, 1989).

To determine whether the response of the embryonic basal forebrain to BMP-9 depends on the stage or extent of maturation, we studied the effects of BMP-9 on ACh content in septal cultures obtained at E11, E14 and E18. Consistent with reported *in vivo* data on the maturation of the basal forebrain cholinergic neurons, the amount of ACh in the control cultures increased with the gestational age between E11 and E18 (Fig. 2b). BMP-9 increased ACh levels in all of these cultures. However the response to BMP-9 was the highest in cells originating from E14 embryos (27-fold increase in ACh content), precisely the time during embryogenesis when cholinergic differentiation peaks ( Schambra, U.B., Sulik, K.K., Petrusz, P., and Lauder, J.M., J.Comp. Neurol. 288, 101-122, 1989) (Fig. 2b). These data show that the responsiveness of the basal forebrain cells to BMP-9 coincides with the development of cholinergic neurons *in vivo*. The data indicates that the rate of maturation of cholinergic neurons may be enhanced by BMP -9 and it is contemplated therefore that BMP-9 most likely acts on cells committed to become cholinergic, or on cells with restricted fates, one of which is the cholinergic neuronal phenotype. Alternatively, cells from E11 and E18 cultures or those in older animals *in vivo* that are less responsive to BMP-9 may produce antagonists that bind, and thereby inactivate, BMP-9, a mode of regulation that has been observed for other BMPs (Piccolo, S., Sasai, Y., Lu, B., and De Robertis, E.M. Cell 86, 589-598, 1996; Zimmerman, L.B., De Jesus-Escobar, J.M., and Harland, R.M. Cell 86, 599-606, 1996; McMahon, J. A., Takada, S., Zimmerman, L. B., Fan, C. M., Harland, R. M., and McMahon, A. P. Genes Dev 12, 1438-1452, 1998; Mabie, P. C., Mehler, M. F., and Kessler, J. A,. J Neurosci 19, 7077-7088, 1999).

### EXAMPLE V

### Increase in Expression

To determine if the BMP-9-induced increase in ACh content and ChAT and VAChT immunostaining were a consequence of increased expression, mRNA levels of ChAT and VAChT, were measured in septal cultures by Northern blot. Total RNA was extracted from the cells using the acid guanidinium thiocyanate (GT)-phenol-chloroform method (Chomczynski, P. and Sacchi, N. Anal.Biochem. 162, 156-159, 1987; Sambrook, J., Fritsch, E.F., and Maniatis, T. Molecular cloning: a laboratory manual. Cold Spring Harbor Laboratory Press, Cold Spring Harbor, NY, 1989). Northern blotting was performed as described (Berse, B. and Blusztajn, J.K. J.Biol.Chem. 270, 22101-22104, 1995). Signal intensities were quantified directly from the blots with a PhosphoImager 400E and ImageQuant software (Molecular Dynamics). To control for total mRNA content and lack of degradation, the blots were stripped and subsequently hybridized with a mouse glyceraldehyde 3-phosphate dehydrogenase (GAPDH) cDNA probe.

The ChAT and VAChT genes reside in an evolutionarily-conserved single genomic locus (Fig. 3a) (Bejanin, S., Cervini, R., Mallet, J., and Berrard, S. J.Biol.Chem. 269, 21944-21947, 1994; Roghani, A. et al. Proc.Natl.Acad.Sci.USA 91, 10620-10624, 1994; Erickson, J.D. et al. J.Biol.Chem. 269, 21929-21932, 1994) an organization that is thought to permit coordinated regulation of their expression (Usdin, T.B., Eiden, L.E., Bonner, T.I., and Erickson, J.D., TINS 18, 218-224, 1995; Berrard, S. et al. J.Neurochem. 65, 939-942, 1995; Berse, B. and Blusztajn, J.K. J.Biol.Chem. 270, 22101-22104, 1995). In the absence of BMP-9, levels of both ChAT and VAChT mRNA were very low or undetectable in cells derived from E14 embryos after four days in culture. However, addition of BMP-9 significantly increased the expression of ChAT and VAChT mRNA (Fig. 3b) indicating that BMP-9 induced the expression of the cholinergic gene locus.

### EXAMPLE VI

### Direct Action of BMP-9

In order to determine if BMP-9 upregulates cholinergic phenotype by acting directly on responsive cells, we determined the effect of this BMP on ChAT and VAChT expression in a murine septal cell line, SN56T17 (Berse, B. and Blusztajn, J.K. J.Biol.Chem. 270, 22101-22104, 1995; Berse, B., López-Coviella, I., and Blusztajn, J.K. Biochem.J. 342, 301-308, 1999). BMP-9 increased the expression of ChAT and VAChT mRNA in these cells (Fig. 3b). Note that this action of BMP-9 was quantitatively smaller in SN56T 17 cells than in primary neurons, presumably because the former are already committed cholinergic cells with high basal expression of ChAT and VAChT. Moreover, BMP-9 increased the abundance of endogenous ChAT mRNA originating from the M promoter (Fig. 3c) and stimulated the expression of a luciferase reporter gene driven by a 4.8 kb fragment of the ChAT gene containing this promoter (Fig. 3a,d). These results show that upregulation of the cholinergic phenotype by BMP-9 occurs in a homogeneous population of competent cells, supporting the notion that in primary brain cultures, BMP-9 also acts directly on cholinergic precursor cells and not by inducing diffusible factors originating from other cell types. Moreover, the data point to the presence of a BMP-9-responsive region within the cholinergic gene locus.

### EXAMPLE VII

### BMP-9 as Maintenance Factor

When cholinergic neurons are deprived of their targets and/or tropic factors they lose their ChAT marker, but do not die Sofroniew, M.V., Galletly, N.P., Isacson, O., and Svendsen, C.N. Science 247, 338-342 1990; Sofroniew, M.V. et al. J.Neurosci. 13, 5263-5276, 1993; Naumann, T., Kermer, P., and Frotscher, M. J.Comp.Neurol. 350, 161-170, 1994). Expression of the cholinergic phenotype is not a fully intrinsic property of these neurons, but rather its maintenance continues to depend on extrinsic signals. To determine if BMP-9 may also act as a maintenance factor for septal cholinergic neurons, the cells were cultured for three days in the presence of BMP-9 (which resulted in a 14 fold increase in ACh content), and subsequently grown in the presence or absence of this BMP for another three day period (Fig. 4a). Cells treated continuously with BMP-9 maintained their high ACh content, while in cells deprived of BMP-9 ACh levels were reduced by 80%, indicating that BMP-9 was necessary for the maintenance of the induced cholinergic phenotype. Significantly, a three-day treatment of the cells with BMP-9 following a deprivation period resulted in a two-fold upregulation of ACh synthesis, indicating that they retain their responsiveness to BMP-9.

### EXAMPLE VIII

### FGF and BMP Interaction Behavior

bFGF and BMP signaling may interact in the development of the nervous system (Mabie, P. C., Mehler, M. F., and Kessler, J.A., J Neurosci 19,7077-7088, 1999; Song, Q.B., Mehler, M.F., and Kessler, J.A., Dev.Biol. 196, 119-127, 1998; Streit, A. and Stem, C. D. Mech Dev 82, 51-66 1999). FGF promotes the proliferation of progenitor cells, preventing their exit from the cell-cycle and contributing to the specification of progenitor cell identity (Ericson, J., Norlin, S., Jessell, T. M., and Edlund, T. Development 125, 1005-1015 1998). BMPs, in contrast, tend to reduce cell proliferation, promoting cell differentiation and cell lineage restriction. These apparently opposing effects may contribute during development to a specific cell fate (Kretzschmar, M., Doody, J., and Massagué, J. Nature 389, 618-622, 1997). To determine if bFGF is necessary for BMP-9 to increase ACh levels, the response of septal cells to BMP-9 in the presence and absence of bFGF was measured. Whereas bFGF had no effect on ACh synthesis by itself, BMP-9 alone was sufficient to increase ACh levels by more than 4-fold as compared to controls (Fig. 4b). However, in the presence of bFGF, the effect of BMP-9 on ACh content was potentiated, and the extent of potentiation by bFGF was concentration-dependent (Fig. 4c). Thus, while BMP-9 is sufficient to induce the cholinergic phenotype, it is more effective in cultures treated with bFGF. These data are consistent with the notion that bFGF stimulates proliferation of cholinergic progenitor cells which are induced by BMP-9 to express the cholinergic phenotype.

The foregoing descriptions detail presently preferred embodiments of the present invention. Numerous modifications and variations in practice thereof are expected to occur to those skilled in the art upon consideration of these descriptions. Those modifications and variations are believed to be encompassed within the claims appended hereto.

### Preferred embodiments of the Invention:

1. A pharmaceutical composition for regulating the cholinergic phenotype of neurons said composition comprising an effective amount of a BMP-9 protein in a pharmaceutically acceptable vehicle.
2. A pharmaceutical composition for differentiating cholinergic neurons, said components comprising an effective amount of BMP-9 in a pharmaceutically acceptable vehicle.
3. A pharmaceutical composition for treating degenerating cholinergic neurons said composition comprising an effective amount of a BMP-9 protein in a pharmaceutically acceptable vehicle.
4. A pharmaceutical composition for treatment of Alzheimer's comprising an effective amount of the composition of item 3.
5. A pharmaceutical composition upregulating the genes for choline acetyltransferase said composition comprising an effective amount of the composition of item1.
6. A pharmaceutical composition for upregulating the genes for vesicular acetylcholine transporter said composition comprising an effective amount of the composition of item 1.
7. A method for differentiating cholinergic neurons in a patient in need of same comprising administering to said patient an effective amount of the composition of item 4.
8. A method for treating Alzheimer's in a patient in need of same comprising:
   a) applying a BMP-9 composition of item 4to embryonic basal forebrain cells to differentiate cholinergic neurons in vitro; and
   b) transplanting said treated cells in said patient.
9. A method for treating degenerating and/or malfunctioning cholinergic neurons in a patient in need of same comprising administering to said patient an effective amount of the composition of item 3.
10. A method for treating degenerating and/or malfunctioning cholinergic neurons in a patient in need of same comprising the steps of
   a) treating embryonic basal forebrain cells in vitro with BMP-9 to generate cholinergic neurons; and
   b) transplanting said treated cells in said patient.
11. A method for upregulating the genes for choline acetyltransferase in a patient in need of some administering a composition of item 5 to said patient.
12. A method for upregulating vesicular acetylcholine transporter in a patient in need of same comprising administering a composition of item 6 to said patient.
13. A method for the differentiation and/or maintenance of cholinergic neurons in a patient in need of same comprising:
   a) transfecting cells with a vector comprising a nucleotide sequence encoding BMP-9; and
   b) implanting said transfected cells in said patient.
14. A method for treating degenerating motor neurons in a patient in need of same comprising administering to said patient an effective amount of a BMP 9 composition.
15. A pharmaceutical composition for regulating the cholinergic phenotype of neurons said composition comprising an effective amount of a BMP selected from the group consisting of BMP-2, BMP-4, BMP-6, BMP-7 and BMP-12.
16. A pharmaceutical composition upregulating the genes for choline acetyltransferase said composition comprising an effective amount of the composition of item 15.

## Claims

1. Use of an effective amount of a BMP-9 protein for the preparation of a medicament for treating degenerating and/or malfunctioning cholinergic neurons in a patient in need of same wherein the medicament is used to upregulate the genes for choline acetyltransferase and/or vesicular acetylcholine transporter to induce the cholinergic phenotype.

2. Use of a transplantable cell transfected with a vector comprising a nucleotide sequence encoding BMP-9 for the preparation of a medicament for treating degenerating and/or malfunctioning cholinergic neurons in a patient in need of same wherein the medicament is used to upregulate the genes for choline acetyltransferase and/or vesicular acetylcholine transporter to induce the cholinergic phenotype.

3. Use of an effective amount of dissociated transplantable embryonic basal forebrain cells treated with BMP-9 in vitro for the preparation of a medicament for treating degenerating and/or malfunctioning cholinergic neurons in a patient in need of same wherein the medicament is used to upregulate the genes for choline acetyltransferase and/or vesicular acetylcholine transporter to induce the cholinergic phenotype.

4. Use of an effective amount of a BMP-9 protein for the preparation of a medicament for treating degenerating motor neurons in a patient in need of same wherein the medicament is used to upregulate the genes for choline acetyltransferase and/or vesicular acetylcholine transporter to induce the cholinergic phenotype.

5. Use according to any of claims 1-4 wherein the medicament is used for treating neuronal diseases and/or defects.

6. Use according to any of claims 1- 3 wherein the medicament is used for treating Alzheimer's disease.

7. Use according to claim 4 wherein the medicament is used for treating amyotrophic lateral sclerosis (ALS).
